Europäisches Patentamt

European Patent Office

Office européen des brevets

(11) Veröffentlichungsnummer: **0 490 381 A1**

# EUROPÄISCHE PATENTANMELDUNG

(21) Anmeldenummer: **91121360.1**

(22) Anmeldetag: **12.12.91**

(51) Int. Cl.5: **C07C 209/70**

(30) Priorität: **14.12.90 DE 4039935**

(43) Veröffentlichungstag der Anmeldung:
**17.06.92 Patentblatt 92/25**

(84) Benannte Vertragsstaaten:
**BE CH DE ES FR GB IT LI NL SE**

(71) Anmelder: **HOECHST AKTIENGESELLSCHAFT**
**Postfach 80 03 20**
**W-6230 Frankfurt am Main 80(DE)**

(72) Erfinder: **Fruth, Anton**
**Fabrikstrasse 37**
**W-8268 Garching/Alz, Hart(DE)**
Erfinder: **Strauss, Julius**
**St.-Stephan-Strasse 29**
**W-8262 Altötting(DE)**
Erfinder: **Stühler, Herbert, Dr.**
**Hochfellnstrasse 12**
**W-8269 Burgkirchen(DE)**

(54) **Verfahren zur Herstellung von gesättigten primären Fettaminen durch Hydrierung der entsprechenden ungesättigten primären Fettamine.**

(57) Beim neuen Verfahren erfolgt die Hydrierung der Doppelbindungen in einem ungesättigten primären Fettamin in der Weise, daß man das ungesättigte primäre Fettamin in flüssiger Phase und in Gegenwart von 0,1 bis 10 Gew.-% Nickel- oder Kobalt-Katalysator mit Wasserstoff bei einer Temperatur von 80 bis 160 °C und einem Druck von 1 bis 40 bar umsetzt. Wenn das zu hydrierende Fettamin Ammoniak enthält, wird es vor dem Hydriervorgang weitgehend entfernt.

EP 0 490 381 A1

Die Erfindung betrifft ein Verfahren zur Herstellung von gesättigten primären Fettaminen durch Hydrierung von ungesättigten primären Fettaminen in flüssiger Phase in Gegenwart von Nickel- oder Kobalt-Katalysatoren.

Die Herstellung von gesättigten primären Fettaminen erfolgt bekanntlich in der Regel durch Hydrierung der entsprechenden gesättigten Fettsäurenitrile. So wird in der US-Patentschrift 3,293,298 ein Verfahren zur Herstellung von gesättigten primären Fettaminen, wie Laurylamin, Stearylamin und Sebacylamin, beschrieben, bei dem das entsprechende Alkylnitril in Gegenwart eines Nickel-Katalysators und in Gegenwart von Ammoniak bei einer Temperatur von 120 bis 150 °C und einem Wasserstoffdruck von 5 bis 25 bar hydriert wird. Der Einsatz einer mehr oder weniger großen Menge an Ammoniak wird für notwendig gehalten, um die Bildung der Nebenprodukte, sekundäres und tertiäres Amin, zurückzudrängen und eine hohe Ausbeute an gesättigtem primärem Fettamin zu erreichen.

Im Falle der Hydrierung von ungesättigten Fettsäurenitrilen zu gesättigten primären Fettaminen mit Nickel- oder Kobalt-Katalysatoren herrscht allgemein die Auffassung vor, daß man neben dem Einsatz von Ammoniak auch einen relativ hohen Druck und/oder eine hohe Temperatur anwenden muß, damit nicht nur die Nitrilgruppe zur primären Aminogruppe, sondern auch die olefinischen Doppelbindungen hydriert werden. Aufgrund des Standes der Technik scheint es also, daß die Hydrierung von olefinischen Doppelbindungen im ungesättigten primären Fettamin sowohl hohe Drücke und/oder hohe Temperaturen als auch die Anwesenheit von relativ viel Ammoniak erfordert.

Die Aufgabe der Erfindung besteht demnach darin, ein Verfahren zur Hydrierung von olefinischen Doppelbindungen in einem ungesättigten primären Fettamin zur Verfügung zu stellen, mit dem diese Hydrierung und damit die Herstellung von gesättigten primären Fettaminen in einfacher Weise in hoher Ausbeute erreicht wird.

Das erfindungsgemäße Verfahren zur Herstellung von gesättigten primären Fettaminen durch Hydrierung von ungesättigten primären Fettaminen in flüssiger Phase in Gegenwart von Nickel- oder Kobalt-Katalysatoren ist dadurch gekennzeichnet, daß das ungesättigte primäre Fettamin mit Wasserstoff in Gegenwart von 0,1 bis 10 Gew.-% Katalysator, vorzugsweise 0,5 bis 5 Gew.-% Katalysator, bezogen auf Fettamin, bei einer Temperatur von 80 bis 160 C, vorzugsweise 100 bis 140 °C, und einem Druck von 1 bis 40 bar, vorzugsweise 1 bis 25 bar, umgesetzt wird.

Es ist überraschend, daß mit dem erfindungsgemäßen Verfahren gesättigte primäre Fettamine in hoher Ausbeute erhalten werden, da doch die Meinung herrscht, daß primäre Fettamine in Gegenwart von Metallen, wie Nickel, Kobalt, Palladium und dergleichen, bereits bei einer Temperatur von etwa 100 °C Ammoniak abspalten und dies nur durch Anwendung eines mehr oder weniger hohen Ammoniak-Partialdruckes verhindert werden kann.

Das Ausgangsmaterial für das erfindungsgemäße Verfahren sind ungesättigte primäre Fettamine mit 8 bis 22 C-Atomen, vorzugsweise 12 bis 18 C-Atomen, oder deren Gemische oder Gemische aus diesen Fettaminen und aus bis zu 90 Gew.-%, vorzugsweise bis zu 60 Gew.-%, gesättigten primären Fettaminen der genannten Kettenlänge, Gewichtsprozente bezogen auf die Gewichtssumme der gesättigten und ungesättigten primären Fettamine. Als Ausgangsmaterial werden also ungesättigte primäre Fettamine mit 8 bis 22 C-Atomen, vorzugsweise 12 bis 18 C-Atomen, und mit vorzugsweise 1 bis 3 (konjugierten oder vorzugsweise isolierten) Doppelbindungen oder deren Gemische eingesetzt. Ebenso geeignet sind auch Gemische aus ungesättigten und gesättigten primären Fettaminen, vorzugsweise solche, die bei der Hydrierung von Fettsäurenitrilen erhalten werden, die ihrerseits aus den Fettsäuren natürlich vorkommender Fette und Öle hergestellt werden, zum Beispiel die primären Fettamingemische, die bei der Hydrierung der Nitrile aus Talgfettsäure, Cocosfettsäure, Palmkernfettsäure, Tranfettsäure, Baumwollsaatölfettsäure, Rübölsäure, Reisölsäure, Sonnenblumenölsäure und Sojaölsäure anfallen. Diese Gemische können neben den ungesättigten primären Fettaminen bis zu 90 Gew.-%, vorzugsweise bis zu 60 Gew.-%, gesättigte primäre Fettamine mit 8 bis 22 C-Atomen, vorzugsweise mit 12 bis 18 C-Atomen, enthalten. Das erfindungsgemäße Verfahren ist also anwendbar auf ungesättigte primäre Fettamine mit 8 bis 22 C-Atomen, vorzugsweise 12 bis 18 C-Atomen, die 0 bis 90 Gew.-%, vorzugsweise 0 bis 60 Gew.-%, gesättigte primäre Fettamine mit 8 bis 22 C-Atomen, vorzugsweise 12 bis 18 C-Atomen, enthalten. Die in Rede stehenden primären Fettamine haben Iodzahlen von etwa von 10 bis 100. So weisen die genannten Talgfettamine gewöhnlich eine Iodzahl von 30 bis 60 auf (die Iodzahl gibt bekanntlich den Verbrauch g Iod pro 100 g Substanz an).

Das mit dem erfindungsgemäßen Verfahren bei Einsatz der genannten primären Fettamine erhaltene Reaktionsprodukt besteht demnach aus gesättigten primären Fettaminen mit 8 bis 22 C-Atomen, vorzugsweise 12 bis 18 C-Atomen, in der Alkylkette (die Alkylketten können gleich oder verschieden sein).

Zur Durchführung des erfindungsgemäßen Verfahrens gibt man das oder die ungesättigten primären Fettamine oder das Gemisch aus ungesättigten und gesättigten primären Aminen in einen Autoklaven, der mit einem Heiz-/Kühlmantel und einem gut wirksamen Rührer ausgestattet ist. Es kann auch ein Reaktor verwendet werden, dessen Inhalt ständig umgepumpt wird. In der Umpumpleitung eingebaut ist ein Wärmetauscher mit Heiz- und Kühlmöglichkeit sowie ein Injektor, der ständig Gas aus dem Reaktor ansaugt. Die Reaktionsgefäße haben zudem Vorrichtungen zum Ein- und Ableiten von Gasen, zum Befüllen und Entleeren sowie zur Kontrolle und Regelung von Druck und Temperatur. Sofern es zweckmäßig ist, wird man das eingesetzte Fettamin in geschmolzenem Zustand vorlegen. In das Reaktionsgefäß gibt man ferner den zur Hydrierung des Amins vorgesehenen Katalysator. Als Katalysatoren eignen sich Nickel- und Kobalt-Katalysatoren, die mit Spuren anderer Metalle, wie zum Beispiel Calcium, Barium, Eisen, Mangan und Molybdän, dotiert sein können und die in Form von Trägerkatalysatoren, insbesonders pulverförmigen, oder in Form von Raney-Katalysatoren eingesetzt werden können. Bei Trägerkatalysatoren eignen sich als Material beispielsweise Aluminiumoxid, Silikagel, Kieselgur und Bimsstein. Bevorzugt werden Nickel-Katalysatoren verwendet, insbesondere in Form von Raney-Nickel. Die genannten Katalysatoren werden in einer Menge von 0,1 bis 10 Gew.-% zugesetzt, vorzugsweise in einer Menge von 0,5 bis 5 Gew.-%, Gewichtsprozente bezogen auf das Gewicht des eingesetzten Fettamins (die genannten Gewichtsprozentmengen beziehen sich klarerweise auf die Elemente Nickel und Kobalt, umfassen also nicht zum Beispiel auch das Trägermaterial).

Es ist vorteilhaft, daß das Ausgangsgemisch im wesentlichen frei von Wasser ist. Die Entfernung eventuell vorhandenen Wassers kann zum Beispiel dadurch erreicht werden, daß man das Ausgangsgemisch (Fettamin und Katalysator) auf eine Temperatur von über 100 °C erhitzt, vorzugsweise auf eine Temperatur von 120 bis 130 °C, und in bekannter Weise mit Stickstoff unter gutem Rühren spült. Analoges gilt auch für den Ammoniakgehalt im Ausgangsgemisch. Primäre Fettamine oder Fettamingemische, die aus der Hydrierung von Fettsäurenitrilen stammen, können bekanntlich eine mehr oder weniger große Menge an Ammoniak enthalten. In diesem Fall ist das primäre Fettamin oder Fettamingemisch zunächst von Ammoniak zu befreien, da es für die erfindungsgemäße Hydrierung im wesentlich ammoniakfrei vorliegen soll. So soll der Ammoniakgehalt höchstens 0,1 mol, vorzugsweise höchstens 0,05 mol, pro mol eingesetztes primäres Fettamin betragen. Es ist also bevorzugt, daß das nach dem erfindungsgemäßen Verfahren zu hydrierende primäre Fettamin oder Fettamingemisch, wenn nicht ganz, so doch praktisch frei von Ammoniak ist.

Die erfindungsgemäße Hydrierung wird bei einem Druck (Gesamtdruck) von 1 bis 40 bar, vorzugsweise 1 bis 25 bar, und einer Temperatur, die je nach Art und Menge des Katalysators und der Art des eingesetzten primären Fettamins oder Fettamingemisches im Temperaturbereich von 80 bis 160 °C liegt, vorzugsweise von 100 bis 140 °C, durchgeführt. Von den angegebenen Druck- und Temperaturwerten wird man die höheren dann wählen, wenn man eine relativ kurze Reaktionszeit erreichen möchte. Mit der Zufuhr von Hydrierwasserstoff in das Reaktionsgefäß kann vor oder nach dem Erhitzen auf die Reaktionstemperatur begonnen werden (es ist zweckmäßig die erste Wasserstoffmenge vor dem Erhitzen zuzusetzen). Der Hydrierwasserstoff kann kontinuierlich oder portionsweise unter Aufrechterhalten der angegebenen Temperatur und des angegebenen Druckes in das Reaktionsgefäß eingebracht werden, wobei klarerweise für einen innigen Kontakt mit dem Reaktionsgemisch gesorgt wird (zum Beispiel durch kräftiges Rühren, ständiges Umpumpen oder Kreisgasfahrweise). Der Hydrierwasserstoff wird in einer solchen Menge und solange zugeführt, bis alle oder im wesentlichen alle ungesättigten Bindungen zu gesättigten hydriert sind (Bestimmung der Iodzahl). Falls die Hydrierung bei Normaldruck, das heißt drucklos, durchgeführt wird, was erfindungsgemäß ebenfalls möglich ist, sollte eventuelles Eindringen von Luft in das Reaktionsgefäß durch entsprechende Maßnahmen verhindert werden. Die Zeit für die erfindungsgemäße Hydrierung umfaßt in der Regel 2 bis 6 Stunden und hängt im wesentlichen von der Katalysatormenge, dem Reaktionsdruck und der Reaktionstemperatur ab.

Nach Abschluß der Hydrierung liegt das angestrebte primäre und gesättigte Fettamin vor. Falls die Abtrennung des eingesetzten Katalysators und die Gewinnung eines katalysatorfreien, gesättigten primären Fettamins gewünscht wird, kann dies zum Beispiel einfach durch Dekantierung oder Filtrierung erreicht werden. Zur weiteren Reinigung kann das Umsetzungsprodukt anschließend noch destilliert werden. Der zurückgewonnene Katalysator ist auch für weitere erfindungsgemäße Hydrierungen geeignet.

Das erfindungsgemäße Verfahren weist eine Reihe von Vorteilen auf. Es ist einfach in der Durchführung. Es liefert die angestrebten primären gesättigten Fettamine mit einer Iodzahl von weniger als 5 und in einer hohen Ausbeute, das heißt ohne eine nennenswerte Menge an den Nebenprodukten sekundäres und tertiäres Fettamin. Die hohe Ausbeute und Reinheit des Fettamins wird in einer relativ kurzen Gesamtreaktionszeit erreicht. Das erfindungsgemäße Verfahren zeichnet sich also auch durch eine hohe Wirtschaftlichkeit aus. Das erhaltene primäre gesättigte Fettamin ist ferner von heller Farbe und behält die gute Farbe zum Beispiel auch im Falle von Alkoxylierungsreaktionen bei. Die in Rede stehenden primären gesättigten Fettamine sind bekanntlich wertvolle Produkte zur Herstellung von Waschmitteln, Herbiziden, Desinfektionsmitteln, Antistatika, Antibackmittel, Textilausrüstungsmitteln und Flotationsmittel.

Die Erfindung wird nun an Beispielen noch näher erläutert.

Beispiel 1

700 g (3,5 mol) primäres Cocosfettamin und 30 g Raney-Nickel (das sind 4,3 Gew.-% Nickel, bezogen auf das Amin) werden in einem 2-l-Rührkolben vorgelegt. Das primäre Cocosfettamin hat eine Iodzahl von 9 und einen Ammoniakgehalt von 0,04 mol pro mol Cocosfettamin.
Zur Hydrierung des primären Cocosfettamins wird der Inhalt des Rührkolbens zunächst mit Stickstoff gespült und dann auf 140 °C erhitzt und bei dieser Temperatur gehalten. Bereits während des Erhitzens wird mit der Zufuhr von Wasserstoff begonnen, und zwar in einer Menge von 140 l Wasserstoff pro Stunde. Diese Wasserstoffzufuhr wird 3 ½ Stunden lang fortgeführt. Nach dieser Zeit ist die bei Normaldruck durchgeführte Hydrierung der Doppelbindungen in der Kohlenstoffkette des primären Cocosfettamins beendet. Es liegt ein praktisch gesättigtes primäres Cocosfettamin vor, wie die Iodzahl von 3 zeigt. Die Ausbeute beträgt 97,0 % der Theorie.

Beispiel 2

800 g (3,2 mol) primäres Oleylamin mit einer Iodzahl von 88 und 40 g Raney-Nickel, das schon dreimal für die gleiche Reaktion eingesetzt war, (das sind 5,0 Gew.-% Nickel, bezogen auf Oleylamin) werden in einem 2-l-Rührautoklaven vorgelegt.
Zur Hydrierung des ammoniakfreien Oleylamins wird der Rührautoklav mit Wasserstoff gespült, und dann wird Wasserstoff bis auf einen Druck von 6 bar aufgedrückt. Nach dem Einschalten des Rührwerks und Erhitzen der Mischung auf 135 °C erfolgt die Hydrierung der Doppelbindungen, indem der verbrauchte Wasserstoff durch eine Druckregelung ständig nachgeführt und der genannte Druck von 6 bar aufrechterhalten wird. Nach 3 ½ Stunden ist die Hydrierung der Doppelbindungen im Oleylrest des primären Oleylamins beendet. Die Ausbeute an gesättigtem primärem Amin (Stearylamin) beträgt 98,0 % der Theorie. Die Iodzahl des Produktes ist 3.

Beispiel 3

35 kg (140 mol) primäres Talgfettamin und 0,88 kg Raney-Nickel, das bereits zweimal für die gleiche Reaktion eingesetzt war, (das sind 2,5 Gew.-% Nickel, bezogen auf Talgfettamin) werden in einem mit Stickstoff gespülten Reaktor vorgelegt. Der Reaktor ist so ausgestattet, daß sein Inhalt mit einer Pumpe über einen Wärmetauscher und einen Injektor zurück in den Reaktor gepumpt werden kann. Der Injektor saugt dabei gleichzeitig Gas aus dem Reaktor an und mischt es intensiv mit der Flüssigkeit. Das Talgfettamin hat eine Iodzahl von 50 und besteht im einzelnen aus 65 Gew.-% primärem Fettamin mit 18 C-Atomen und 35 Gew.-% primärem Fettamin mit im wesentlichen 14 und 16 C-Atomen, wobei 50 Gew.-% ungesättigtes primäres Fettamin mit 1 bis 3 Doppelbindungen und 50 Gew.-% gesättigtes primäres Fettamin sind. Es enthält ferner 0,05 mol Ammoniak pro mol Talgfettamin.
Zur Hydrierung wird die Reaktorpumpe eingeschaltet und die Reaktionsmischung auf 130 °C erhitzt. Bei dieser Temperatur wird Wasserstoff bis auf einen Druck von 15 bar aufgedrückt, und dieser Druck wird bei der genannten Temperatur von 130 °C durch ständige Wasserstoffzugabe gehalten. Nach 3 Stunden ist die Hydrierung der Doppelbindungen abgeschlossen. Es liegt ein gehärtetes primäres Talgfettamin mit einer Iodzahl von 4 in einer Ausbeute von 98,0 % der Theorie vor.

Beispiel 4

35 kg (140 mol) primäres Sojafettamin und 0,35 kg Raney-Nickel (das sind 1,0 Gew.-% Nickel, bezogen auf Amin) werden in dem mit Stickstoff gespülten Reaktor des Beispiels 3 vorgelegt. Das Sojafettamin hat eine Iodzahl von 110 und einen Ammoniakgehalt von 0,05 mol pro mol Amin und besteht im einzelnen aus 15 Gew.-% $C_{16}$-Alkylamin, 80 Gew.-% $C_{18}$-Alkylamin und 5 Gew.-% $C_{14}$-, $C_{20}$- und $C_{22}$-Alkylamin, wobei 75 Gew.-% des $C_{18}$-Alkylamins 1, 2 oder 3 Doppelbindungen enthalten.
Zur Hydrierung wird die Reaktorpumpe eingeschaltet und die Reaktionsmischung auf 105 °C erhitzt und bei dieser Temperatur gehalten. Bereits während des Erhitzens wird Wasserstoff bis auf einen Druck von 25 bar aufgedrückt, und dieser Wasserstoffdruck wird aufrechterhalten, bis kein Druckabfall mehr feststellbar ist, was nach 5 ½ Stunden der Fall ist. Es liegt ein gehärtetes primäres Sojafettamin mit einer Iodzahl von 2 in einer Ausbeute von 97,0 % der Theorie vor.

**Patentansprüche**

1.　Verfahren zur Herstellung von gesättigten primären Fettaminen durch Hydrierung von ungesättigten primären Fettaminen in flüssiger Phase in Gegenwart von Nickel- oder Kobalt-Katalysatoren, dadurch gekennzeichnet, daß das ungesättigte primäre Fettamin mit Wasserstoff in Gegenwart von 0,1 bis 10 Gew.-% Katalysator, bezogen auf Fettamin, bei einer Temperatur von 80 bis 160 °C und einem Druck von 1 bis 40 bar, umgesetzt wird.

2. Verfahren nach Anspruch 1, wobei das ungesättigte primäre Fettamin mit Wasserstoff in Gegenwart von 0,5 bis 5 Gew.-% Katalysator, bezogen auf Fettamin, bei einer Temperatur von 100 bis 140 °C und einem Druck von 1 bis 25 bar umgesetzt wird.

3. Verfahren nach Anspruch 1 oder 2, wobei Nickel-Katalysatoren eingesetzt werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 003 933 (CH.A. DRAKE)<br>* Spalte 2, Zeile 24 - Zeile 30 *<br>* Spalte 6, Zeile 23 - Zeile 59 *<br>--- | 1-5 | C07C209/70 |
| X | GB-A-2 025 408 (THE LION FAT & OIL CO.)<br>* Seite 3, Zeile 58 - Zeile 88 *<br>--- | 1-5 | |
| A | US-A-3 896 173 (CH.A. DRAKE)<br>* Spalte 1, Zeile 32 - Zeile 42 *<br>* Spalte 6, Zeile 57 - Zeile 65 *<br>* Spalte 9, Zeile 37 - Zeile 49 *<br><br>----- | 1-5 | |
| | | | **RECHERCHIERTE SACHGEBIETE (Int. Cl.5)**<br><br>C07C |

Der vorliegende Recherchenbericht wurde für alle Patentansprüche erstellt

| Recherchenort | Abschlußdatum der Recherche | Prüfer |
|---|---|---|
| DEN HAAG | 19 MAERZ 1992 | PAUWELS G. R. A. |